# EUROPEAN PATENT SPECIFICATION

(11) **EP 2 228 062 B1**
(45) Date of publication and mention of the grant of the patent: **26.07.2017**
(21) Application number: 10155685.0
(22) Date of filing: 05.03.2010
(51) Int. Cl.: A61K 31/121, A61K 31/4525, A61K 31/685, A61K 31/385, A61P 43/00

(54) **Compositions containing a phospholipid-curcumin complex and piperine as chemosensitizing agent**
Zusammensetzungen, die einen Phospholipid-Curcumin-Komplex und Piperine enthalten, als chemosensibilisierende Mittel
Compositions comprenant un complexe de phospholipide-curcumin et de la pipérine en tant qu'agent chimiosensibilisateur

(30) Priority: 11.03.2009 IT MI20090356
(43) Date of publication of application: 15.09.2010
(73) Proprietor: Velleja Research SRL, 29010 Pontenure (PC) (IT)
(72) Inventor: Di Pierro, Francesco, 29010, Pontenure (PC) (IT)
(74) Representative: Minoja, Fabrizio

(56) References cited:
- EP-A- 1 837 030
- ANTONY B ET AL: "A Pilot Cross-Over Study to Evaluate Human Oral Bioavailability of BCM-95 (R) CG (Biocurcumax (TM)), A Novel Bioenhanced Preparation of Curcumin" INDIAN JOURNAL OF PHARMACEUTICAL SCIENCES, vol. 70, no. 4, July 2008 (2008-07), pages 445-449, XP008114578 ISSN: 0250-474X
- ANAND PREETHA ET AL: "Bioavailability of curcumin: Problems and promises" MOLECULAR PHARMACEUTICS, vol. 4, no. 6, November 2007 (2007-11), pages 807-818, XP008114486 ISSN: 1543-8384
- CHEARWAE W ET AL: "Biochemical mechanism of modulation of human P-glycoprotein (ABCB1) by curcumin I, II, and III purified from Turmeric powder" BIOCHEMICAL PHARMACOLOGY, PERGAMON, OXFORD, GB, vol. 68, no. 10, 15 November 2004 (2004-11-15), pages 2043-2052, XP004598336 ISSN: 0006-2952
- HOUR TZYH-CHYUAN ET AL: "Curcumin enhances cytotoxicity of chemotherapeutic agents in prostate cancer cells by inducing p21WAFI/CIPI and C/EBPbeta expressions and suppressing NF-kappaB activation" PROSTATE, vol. 51, no. 3, 15 May 2002 (2002-05-15), pages 211-218, XP002554164 ISSN: 0270-4137

## Description

The present invention relates to compositions containing curcumin in phospholipid-complexed form and piperine with chemosensitising action for the treatment of drug resistance.

### PRIOR ART

The activity of many medicaments is known to depend on their ability to cross biological barriers (mucous membranes, epithelia, plexi and membranes) to reach their target. Medicaments with a high lipophilic content are able to cross these barriers in the absence of specialised transport systems, by means of passive diffusion through the cytoplasmic, nuclear and mitochondrial membranes. Conversely, highly hydrophilic compounds require specific transport mechanisms to facilitate their transport.

In practice, the factor which limits the quantity of a medicament that reaches a given target is not so much its ability to enter the cell, but its tendency to leave it. This phenomenon depends on pharmacological extrusion mechanisms present in plasma membranes, which perform a critical role by limiting the absorption and accumulation of exogenous substances in the cells and thus making them resistant to various medicaments. This phenomenon, called MDR (Multidrug Resistance) is particularly evident in tumour cells which, when they become MDR +, acquire the ability to extrude compounds with antitumoral action. The molecules responsible for Multidrug Resistance are carriers with an ATP-dependent protein structure encoded by ABC genes (ATP-binding cassette). ABC carriers play a physiological role of detoxification/protection of the body from xenobiotic substances, by means of one-way transport of compounds from the cytoplasm to an intracellular compartment (endoplasmatic reticulum, mitochondria, peroxisomes, etc.) or to the extracellular space outside the cell, for subsequent elimination by the body through the urine and/or bile. At least six forms of the ABC genes are associated with extrusion of antitumoral medicaments. In particular, gene ABCB1, also known as the multidrug resistance (*MDR1*) gene, is the transporter best characterised in biochemical terms. It encodes for a 170 KDa glycoprotein-P (gp-P) which plays an important role in the development of resistance to many antitumoral chemotherapy drugs, precluding their accumulation in tumour cells.

Extrusion via gp-P is described for the following antitumorals: actinomycin D, daunorubicin, doxorubicin, etoposide, mitomycin C, paclitaxel, tamoxifen, topotecan, vinblastine and vincristine. However, its involvement cannot be ruled out in the case of many other antitumorals. Other classes of medicaments also constitute a substrate for gp-P: they include calcium antagonists like diltiazem, nicardipine and verapamil; cardiotonics like propafenone, amiodarone, quinidine, and digoxin; morphine-6-glucuronide and loperamide; protease inhibitors such as indinavir, ritonavir and saquinavir; immunosuppressants such as cyclosporin A and tacrolimus; drugs which act on the central nervous system such as domperidone, fluphenazine, ondansetron, perphenazine, phenoxazine and phenytoin; and steroids such as aldosterone, dexamethasone, hydrocortisone and others.

The majority of chemosensitising agents present an intrinsic degree of toxicity which limits their use.

A potent chemosensitising drug with low toxicity is curcumin, chemically diferuloylmethane, obtained by extraction from *Curcuma longa,* up to degrees of purity of between 95 and 99%. Numerous *in vitro* studies have demonstrated the ability of curcumin to revert the MDR of numerous cell lines of murine and human origin, thus acting as a chemosensitising agent (Angelini A et al. Oncol Rep, 2008; 20(4): 731-735*).*

However, curcumin presents the drawback of poor intestinal absorption (Anand P et al. Mol Pharm, 2007; 4(6): 807-818*),* rapid microsomal inactivation with glucuronidation and/or sulphation followed by rapid elimination (Vareed SK et al. Cancer Epidemiol Biomarkers Prev, 2008; 17(6): 1411-1417*);* and rapid metabolisation (Aggarwal BB et al. Trends Pharmacol Ski, 2009; 30(2): 85-94*)* to tetra-hydrocurcumin, hexa-hydrocurcumin and octa-hydrocurcumin when administered systemically (i.v. and i.p.). The low oral bioavailability of curcumin, and its high rate of inactivation through the microsomal enzymes, causes the substantial absence of plasma detection after oral administration, even at doses of up to 12 g/person.

In an attempt to overcome said limitations, some authors have found that the simultaneous administration of curcumin and piperine (an alkaloid obtained by extraction from *Piper nigrum*), both pure, determines a 2000% increase in oral bioavailability, namely an approximately 20-fold increase in oral bioavailability compared with administration of pure curcumin (Shoba G et al. Planta Med, 1998; 64(4): 353-356*).*

EP 810868B1 discloses the action of piperine enhancers in improving the bioavailability of nutrients through absorption at intestinal level. That document does not clarify the action mechanism, but merely lists a series of hypotheses, including inhibitory action exerted on the microsomal detoxification enzymes, and an effect on intestinal absorption capacity. This last hypothesis, however, was rejected by a recent study (Bansal T et al, 2008; Life Sci 83(7-8): 250-259*),* which totally rules out the ability of piperine to influence intestinal absorption processes. This hypothesis is also indirectly demonstrated by the results obtained by administering curcumin in phytosome form, in the absence of piperine, reported later in the description, in the section relating to pharmacological tests.

### DESCRIPTION OF THE INVENTION

It has now surprisingly been found that the simultaneous administration of curcumin in distearoylphosphatidylcholine-complexed form (hereinafter phospholipid-complexed form) and pure piperine increases the plasma concentration of curcumin approximately 260-fold compared with the concentration obtained after administration of the same amount of pure curcumin, not complexed and not associated with piperine.

It is therefore evident that the simultaneous administration of distearoylphosphatidylcholine-complexed -complexed curcumin and pure piperine increases the oral bioavailability of curcumin to a much greater extent than the prior art, where the combination of non-complexed curcumin and piperine produced a plasma concentration 20 times greater than the administration of curcumin alone (see Shoba G et al., reported above). Such a great increase was not foreseeable, and cannot be explained solely in terms of increased bioavailability of curcumin, but must be attributed to other, possibly synergistic mechanisms. To sum up, the administration of pure curcumin produces a scarcely detectable plasma curcumin concentration (at doses of up to 12g/person); the association of the same curcumin with pure piperine produces a plasma increase approx. 20 times greater than the value obtained with curcumin alone; simultaneous administration of distearoylphosphatidylcholine- -complexed curcumin and pure piperine produces a plasma increase approx. 260 times greater than the value obtained with the administration of curcumin alone.

The high plasma concentration of curcumin thus obtained causes the chemosensitisation of various neoplastic forms, and reverts their drug resistance. It also allows the use of lower doses of chemotherapy drugs, thus reducing their toxic side effects.

The present invention therefore relates to compositions containing in distearoylphosphatidylcholine-complexed curcumin and pure piperine with chemosensitising action for the treatment of drug resistance, in particular drug resistance induced by chemotherapy drugs.

According to the invention, curcumin will be present in the form of complexes with distearoylphosphatidylcholine as described, for example, in EP 441 279. Phospholipid-complexed forms, available commercially under the name of Phytosome®, present a documented action as enhancers of the bioavailability of the active ingredient carried. However, as already stated, said enhancing activity alone is insufficient to explain the very large increase in bioavailability demonstrated by the compositions according to the invention.

According to the invention, the term "piperine" in the description always means piperine in pure form.

The compositions of the invention will preferably contain curcumin phytosome and piperine in the ratio of between 100:1 and 1:1; curcumin phytosome will be present in the amount of between 12 and 0.4 g, and piperine will be present in the amount of between 120 and 4 mg.

According to a particularly preferred aspect, the compositions of the invention will contain curcumin phytosome and piperine in the ratio of 100:1, curcumin phytosome will be present in the amount of 400 mg, and piperine will be present in the amount of 4 mg.

The compositions of the invention may be administered orally or parenterally.

In the case of the oral administration, the doses may be divided into between 1 and 12 daily administrations (in the latter case every 1.5 hours). The oral compositions will preferably be in solid form, and could possibly be formulated in time- and/or pH-dependent modified-release forms.

In the case of parenteral administration, the compositions of the invention could be formulated as liquids for intravenous infusion.

The compositions of the invention could also contain antioxidant and/or tissue-protecting ingredients, in particular central nervous system protectors, to prevent the risk of neurotoxicity resulting from the chemosensitisation caused by curcumin. Moreover, in view of its high presence in the plasma, curcumin may reduce the protection provided by the blood-brain barrier and other biological barriers (testicular, placental, etc.), which is also effected by the drug-extruding action of Gp-P.

According to a preferred aspect, the compositions of the invention will contain, in addition to curcumin phytosome and piperine, substances such as glutathione (1-2000 mg/dose; preferably 100 mg), lipoic acid (1-1600 mg; preferably 200 mg); ubidecarenone (1-500 mg; preferably 50 mg); vine polyphenols, bilberries and cranberries, milk thistle, green tea, ginkgo in free or phytosome form or otherwise complexed (1-2000 mg: preferably 200 mg).

### PHARMACOLOGICAL TESTS

The oral bioavailability of curcumin, detected as total plasma curcumin, was evaluated after administration in pure form, either alone or in phytosome form combined with pure piperine.

For this purpose, three groups of healthy volunteers (10 individuals per group) were treated orally with:

| | |
|---|---|
| Test group: | 400 mg of curcumin phytosome combined with 4 mg of piperine. |
| Control group a): | 400 mg of pure curcumin. |
| Control group b): | 4000 mg of pure curcumin. |

### Measurement of the areas under the curve (AUC) gave the following results:

| | |
|---|---|
| Test group: | AUC of 596080±29872; |
| Control group a): | AUC of 2240±197; |
| Control group b): | AUC of 23800±1212. |

The data set out above demonstrate that simultaneous administration of curcumin phytosome combined with pure piperine increases the oral bioavailability of curcumin approximately 260-fold compared with administration of the same amount of pure curcumin, not complexed and not combined with piperine.

The oral bioavailability of curcumin was also evaluated after administration of curcumin in phytosome form but in the absence of pure piperine.

400 mg of curcumin phytosome without piperine was administered orally to the healthy volunteers. The AUC value obtained was 24404±1857, namely a similar value to that obtained after administration of 4000 mg of pure curcumin without piperine reported in the preceding test.

It was also observed that when curcumin phytosome and pure piperine are administered orally in the preferential ratio of 100:1, at standard doses for curcumin phytosome of between 400 and 4000 mg/dose and for piperine of between 4 and 40 mg/dose, 1 to 12 times a day at 1-1.5 hour intervals, the plasma concentration of curcumin induces chemosensitisation of various neoplastic forms, reverting their drug resistance.

These results were obtained regardless of whether the resistance was identified as intrinsic to the tumour or acquired by the tumour following continual cycles of chemotherapy.

The compositions of the invention will consequently be associated with possible chemotherapy protocols based on antitumorals for which the possibility of resistant tumours is known, because they consist of MDR+ cells.

Some examples of chemotherapy protocols with which the compositions of the invention can be associated are set out below.
1) Treatment of sarcoma of the soft tissues with 3-week cycles based on doxorubicin 45-60 mg/m² i.v., dacarbazine 200-250 mg/m² i.v. and cyclophosphamide 450-500 mg/m² i.v..
2) Treatment of Hodgkin's lymphoma with 28-day cycles (6 cycles) based on doxorubicin 25 mg/m² i.v., bleomycin 10 U/m² i.v., vinblastine 6 mg/m² i.v. and dacarbazine 150 mg/m² i.v.
3) Treatment of pulmonary microcytoma with 3-week cycles based on cyclophosphamide 1000 mg/m² i.v., doxorubicin 45 mg/m² i.v. and etoposide 50 mg/m² i.v or vincristine 1 mg/m² i.v..
4) Treatment of acute lymphoblastic leukaemia with cyclophosphamide 1200 mg/m² i.v, daunorubicin 45 mg/m² i.v., vincristine 2 mg/m² i.v., prednisone 60 mg/m² p.o. and L-asparaginase 6000 U/m² i.v.
5) Treatment of ovarian carcinoma with 21-day cycles based on doxorubicin 50 mg/m² i.v. and cisplatin 50 mg/m² i.v.
6) Treatment of testicular carcinoma with 21-day cycles based on etoposide 100 mg/m² i.v., cisplatin 20 mg/m² i.v. and bleomycin 30 U i.v.
7) Treatment of bladder carcinoma with 21-28 day cycles based on cyclophosphamide 650 mg/m² i.v., doxorubicin 50 mg/m² i.v. and cisplatin 100 mg/m² i.v.
8) Treatment of colorectal carcinoma with up to 1-year cycles based on levamisole 150 mg/day p.o. and 5-fluorouracil 450 mg/m² i.v. or irinotecan 125 mg/m² i.v.
9) Treatment of carcinoma of the CNS with 6-8 week cycles based on lomustine 110 mg/m² i.v., procarbazine 60 mg/m² p.o. and vincristine 1.4 mg/m² i.v.
10) Treatment of breast cancer with 21-day cycles based on paclitaxel 175 mg/m² i.v.

Administration of the compositions of the invention allows the dose of the chemotherapy drugs used to be reduced by a factor of up to 10. In fact, chemosensitisation enhances the action of antitumorals.

The compositions of the invention can be formulated in a way suitable for oral or parenteral administration, and will be prepared according to conventional methods well known in pharmaceutical technology, such as those described in "Remington's Pharmaceutical Handbook", Mack Publishing Co., N.Y., USA, using conventional excipients. In particular, the compositions of the invention could be formulated for oral administration as capsules; normal, multi-layer, differentiated release or gastroprotected tablets; suspensible or effervescent powder; orodispersible powders or suspensions; and for parenteral administration as solutions or suspensions for sterile, pyrogen-free infusion.

Some formulation examples are set out below.

### 1 - FILM-COATED TABLETS

| | |
|---|---|
| Curcumin phytosome | 400 mg |
| Piperine | 4 mg |
| Microcel | 200 mg |
| Dicafos | 200 mg |
| Vegetable magnesium stearate | 40 mg |
| PVP CL | 40 mg |
| Silicon dioxide | 20 mg |
| Shellac | 50 mg |

### 2 - FILM-COATED TABLETS

| | |
|---|---|
| Curcumin phytosome | 400 mg |
| Piperine | 4 mg |
| Lipoic acid | 200 mg |
| Microcel | 200 mg |
| Dicafos | 200 mg |
| Vegetable magnesium stearate | 40 mg |
| PVP CL | 40 mg |
| Silicon dioxide | 20 mg |
| Shellac | 50 mg |

### 3 - CAPSULES FORMAT "0"

| | |
|---|---|
| Curcumin phytosome | 400 mg |
| Piperine | 4 mg |
| Talc | 10 mg |
| Microcel 101 | 50 mg |

### 4 - CAPSULES FORMAT "0"

| | |
|---|---|
| Curcumin phytosome | 400 mg |
| Piperine | 4 mg |
| Lipoic acid | 200 mg |
| Talc | 10 mg |
| Microcel 101 | 50 mg |

### 5 - SACHETS

| | |
|---|---|
| Curcumin phytosome | 800 mg |
| Piperine | 8 mg |
| Lipoic acid | 400 mg |
| Fructose | 1000 mg |
| Methocel E5 | 20 mg |
| Aerosil | 50 mg |
| Acesulfame K sweetener | 10 mg |
| E110 | 2 mg |
| Citrus fruit flavouring | 150 mg |

### 6 - SACHETS

| | |
|---|---|
| Curcumin phytosome | 1600 mg |
| Piperine | 16 mg |
| Saccharose | 2265 mg |
| Citric acid | 50 mg |
| Silicon dioxide | 20 mg |
| Flavouring | 150 mg |
| Acesulfame K | 15 mg |

### 7 - TWO-LAYER CONTROLLED-RELEASE TABLETS

| *Normal-release* layer | |
|---|---|
| Curcumin phytosome | 200 mg |
| Piperine | 2 mg |
| Dicafos | 304 mg |
| Aerosil | 3 mg |
| Vegetable magnesium stearate | 6 mg |
| Colouring | 1 mg |

| *Slow-release* layer | |
|---|---|
| Curcumin phytosome | 400 mg |
| Piperine | 4 mg |
| Metholose | 80 mg |
| Aerosil | 2 mg |
| Vegetable magnesium stearate | 3 mg |
| Microcel | 80 mg |
| Dicafos | 164 mg |

### 8 - ORODISPERSIBLE FORMULATION

| | |
|---|---|
| Curcumin phytosome | 800 mg |
| Piperine | 8 mg |
| Sorbitol | 160 mg |
| Orange flavouring | 20 mg |
| Mandarin flavouring | 5 mg |
| Acesulfame K | 2 mg |
| Aerosil | 5 mg |
| Fructose | 1566 mg |

### 9 - STERILE PYROGEN-FREE INJECTABLE SOLUTION

### (reconstituted to 400 ml)

| | |
|---|---|
| Curcumin phytosome | 1 mg/ml |
| Piperine | 0.01 mg/l |
| Lipoic acid | 0.5 mg/ml |
| Glucose | 5% |
| Ethanol | 1% |
| Sterile, pyrogen-free water | q.s. |

## Claims

1. Compositions containing curcumin in distearoylphosphatidylcholine-complexed form and piperine.

2. Compositions as claimed in claim 1, containing piperine in the pure form.

3. Compositions as claimed in the preceding claims, containing curcumin in distearoylphosphatidylcholine-complexed form and piperine in a ratio ranging from 100:1 to 1:1.

4. Compositions as claimed in claim 3, containing curcumin in distearoylphosphatidylcholine-complexed form and piperine in a 100:1 ratio.

5. Compositions as claimed in the preceding claims, containing curcumin in distearoylphosphatidylcholine-complexed form in amounts ranging from 12 to 0.4 g and piperine in amounts ranging from 120 to 4 mg.

6. Compositions as claimed in claim 5, containing curcumin in distearoylphosphatidylcholine-complexed form in the amount of 400 mg and piperine in the amount of 4 mg.

7. Compositions as claimed in the preceding claims, for oral or parenteral administration.

8. Compositions as claimed in claim 7, in the form of tablets, capsules, sachets, controlled-release forms or orodispersible forms.

9. Compositions as claimed in claim 7, for intravenous infusion.

10. Compositions as claimed in the above claims, also containing substances selected from glutathione, lipoic acid, ubidecarenone, vine polyphenols, bilberry, cranberry, milk thistle, green tea, gingko in the free or complexed forms.

11. A combination of curcumin in distearoylphosphatidylcholine-complexed form and pure piperine for use as chemosensitising compositions in the treatment of drug resistance.

12. A combination for use according to claim 11 further containing a neuroprotective/antioxidant agents.

13. A combination for use according to claim 11 and 12 wherein the neuroprotective/antioxidant agent is lipoic acid in the pure form.

## Patentansprüche

1. Zusammensetzungen, enthaltend Curcumin in Distearoylphosphatidylcholin-komplexierter Form und Piperin.

2. Zusammensetzungen, wie sie in Anspruch 1 beansprucht sind, die Piperin in reiner Form enthalten.

3. Zusammensetzungen, wie sie in den vorangehenden Ansprüchen beansprucht sind, enthaltend Curcumin in Distearoylphosphatidylcholin-komplexierter Form und Piperin in einem Verhältnis, das von 100:1 bis 1:1 reicht.

4. Zusammensetzungen, wie sie in Anspruch 3 beansprucht sind, enthaltend Curcumin in Distearoylphosphatidylcholinkomplexierter Form und Piperin in einem Verhältnis von 100:1.

5. Zusammensetzungen, wie sie in den vorangehenden Ansprüchen beansprucht sind, enthaltend Curcumin in Distearoylphosphatidylcholin-komplexierter Form in Mengen im Bereich von 12 bis 0,4 g und Piperin in Mengen im Bereich von 120 bis 4 mg.

6. Zusammensetzungen, wie sie in Anspruch 5 beansprucht sind, die Curcumin in Distearoylphosphatidylcholinkomplexierter Form in der Menge von 400 mg und Piperin in der Menge von 4 mg enthaltend.

7. Zusammensetzungen, wie sie in den vorangehenden Ansprüchen beansprucht sind, zur oralen oder parenteralen Verabreichung.

8. Zusammensetzungen, wie sie in Anspruch 7 beansprucht sind, in der Form von Tabletten, Kapseln, Sachets, in Formen zur kontrollierten Freisetzung oder orodispergierbaren Formen.

9. Zusammensetzungen, wie sie in Anspruch 7 beansprucht sind, zur intravenösen Infusion.

10. Zusammensetzungen, wie sie in den obigen Ansprüchen beansprucht sind, die auch Substanzen, ausgewählt aus Glutathion, Liponsäure, Ubidecarenon, Weinpolyphenolen, Heidelbeere, Cranberry, Mariendistel, grünem Tee, Gingko, in freier oder komplexierter Form, enthalten.

11. Kombination aus Curcumin in Distearoylphosphatidylcholinkomplexierter Form und reinem Piperin zur Verwendung als chemosensibilisierende Zusammensetzungen in der Behandlung von Arzneimittelresistenz.

12. Kombination zur Verwendung gemäß Anspruch 11, die außerdem ein neuroprotektives/Antioxidans-Mittel enthält.

13. Kombination zur Verwendung gemäß Anspruch 11 oder 12, wobei das neuroprotektive/Antioxidans-Mittel Liponsäure in reiner Form ist.

## Revendications

1. Compositions contenant de la curcumine sous une forme complexée à la distéaroylphosphatidylcholine et de la pipérine.

2. Compositions telles que revendiquées selon la revendication 1, contenant de la pipérine sous la forme pure.

3. Compositions telles que revendiquées selon les revendications précédentes, contenant de la curcumine sous une forme complexée à la distéaroylphosphatidylcholine et de la pipérine sous un rapport s'étendant de 100:1 à 1:1.

4. Compositions telles que revendiquées selon la revendication 3, contenant de la curcumine sous une forme complexée à la distéaroylphosphatidylcholine et de la pipérine en un rapport de 100:1.

5. Compositions telles que revendiquées selon les revendications précédentes, contenant de la curcumine sous une forme complexée à la distéaroylphosphatidylcholine en des quantités s'étendant de 12 à 0,4 g et de la pipérine en des quantités s'étendant de 120 à 4 mg.

6. Compositions telles que revendiquées selon la revendication 5, contenant de la curcumine sous une forme complexée à la distéaroylphosphatidylcholine en la quantité de 400 mg et de la pipérine en la quantité de 4 mg.

7. Compositions telles que revendiquées selon les revendications précédentes, pour l'administration par voie orale ou parentérale.

8. Compositions telles que revendiquées selon la revendication 7, se présentant sous la forme de comprimés, de capsules, de sachets, sous des formes à libération contrôlée ou des formes orodispersibles.

9. Compositions telles que revendiquées selon la revendication 7, pour la perfusion intraveineuse.

10. Compositions telles que revendiquées selon les revendications ci-dessus, contenant également des substances sélectionnées parmi la glutathione, l'acide lipoïque, l'ubidécarénone, les polyphénols de raisin, le bleuet, la canneberge, le chardon-marie, le thé vert, le ginkgo sous des formes libres ou complexées.

11. Combinaison de curcumine sous une forme complexée à la distéaroylphosphatidylcholine et de la pipérine pure pour l'utilisation comme compositions de chimiosensibilisation dans le traitement de la résistance médicamenteuse.

12. Combinaison pour l'utilisation selon la revendication 11 contenant en outre des agents neuroprotecteurs/antioxydants.

13. Combinaison pour l'utilisation selon la revendication 11 et 12, où l'agent neuroprotecteur/antioxydant est l'acide lipoïque sous la forme pure.
